Europäisches Patentamt

European Patent Office

Office européen des brevets

(19)

(11) Publication number: **0 306 181 B1**

(12) # EUROPEAN PATENT SPECIFICATION

(45) Date of publication of patent specification: **16.09.92**

(51) Int. Cl.5: **C01B 33/34**, B01J 29/28, C07C 2/66, C07C 4/18

(21) Application number: **88307651.5**

(22) Date of filing: **18.08.88**

(54) **Two stage synthesis of zeolite.**

(30) Priority: **31.08.87 US 91612**
**31.08.87 US 91613**

(43) Date of publication of application:
**08.03.89 Bulletin 89/10**

(45) Publication of the grant of the patent:
**16.09.92 Bulletin 92/38**

(84) Designated Contracting States:
**AT BE DE FR GB IT NL SE**

(56) References cited:
**EP-A- 0 220 893**
**FR-A- 2 313 980**
**FR-A- 2 429 041**
**US-A- 4 016 245**

(73) Proprietor: **MOBIL OIL CORPORATION**
**3225 Gallows Road**
**Fairfax, Virginia 22037-0001(US)**

(72) Inventor: **Chu, Pochen**
**1173 Ollerton Road**
**West Deptford, NJ 08066(US)**
Inventor: **Klocke, Donald Joseph**
**209 Roberts Drive**
**Somerdale, NJ 08083(US)**
Inventor: **Kirker, Garry Wayne**
**18 Old Mill Road**
**Sewell, NJ 08080(US)**
Inventor: **McWilliams, John Paul**
**117 S. American Street**
**Woodbury, NJ(US)**
Inventor: **Marler, David Owen**
**801 Cooper Street, No. 272 B**
**Deptford, NJ 08096(US)**
Inventor: **Vartuli, James Clarke**
**302 Ponds Edge Road**
**West Chester,PA 19380(US)**

(74) Representative: **Jones, Alan John et al**
**CARPMAELS & RANSFORD 43 Bloomsbury**
**Square**
**London, WC1A 2RA(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to an improved, temperature-programmed method for preparing zeolite ZSM-23 whereby synthesis is facilitated and reproducible, and products exhibiting high purity, small, uniform crystal size and improved catalytic activity and stability can be obtained.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by x-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of large dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as rigid three-dimensional frameworks of $SiO_4$ and $AlO_4$ in which the tetrahedra are cross-linked by the sharing of oxygen atoms whereby the ratio of the total aluminium and silicon atoms to oxygen atoms is 1:2. The electrovalence of the tetrahedra containing aluminium is balanced by the inclusion in the crystal of a cation, for example, an alkali metal or an alkaline earth metal cation. This balanced electrovalence can be expressed by a formula wherein the ratio of aluminium to the number of various cations, such as Ca/2, Sr/2, Na, K or Li is equal to unity. One type of cation may be exchanged either entirely or partially with another type of cation utilizing ion exchange techniques in a conventional manner. By means of such cation exchange, it has been possible to vary the properties of a given aluminosilicate by suitable selection of the cation. The spaces between the tetrahedra are occupied by the molecules of water prior to dehydration.

Prior art techniques have resulted in the formation of a great variety of synthetic zeolites, such as zeolite A (US-A-2,882,243), zeolite X (US-A-2,882,244), zeolite Y (US-A-3,130,007), zeolite ZK-5 (US-A-3,247,195), zeolite ZK-4 (US-A-3,314,752), zeolite ZSM-5 (US-A-3,702,886), zeolite ZSM-11 (US-A-3,709,979) and zeolite ZSM-12 (US-A-3,832,449), merely to name a few.

Although the term "zeolites" encompasses materials containing silica and alumina, it is recognised that silica and alumina may be replaced in whole or in part with other oxides. More particularly, $GeO_2$ is an art-recognised substitute for $SiO_2$. Also, $B_2O_3$, $Cr_2O_3$, $Fe_2O_3$, and $Ga_2O_3$ are art-recognised replacements for $Al_2O_3$. Accordingly, the term "zeolite" as used herein connotes not only materials containing silicon and, optionally, aluminium atoms in the crystalline lattice structure thereof, but also materials which contain suitable replacement atoms for such silicon and/or aluminium. Such replacement, of course, is not necessarily total. On the other hand, the term "aluminosilicate zeolite" as used herein denotes zeolite materials consisting essentially of silicon and aluminium atoms in the crystalline lattice structure thereof.

The present invention is particularly concerned with a zeolite, e.g. alumino-silicates or like material, whose synthesis is conventionally accomplished by creating an aqueous reaction mixture comprising as required components an organic directing agent and sources of alkali metal cations, silica and alumina, and heating the mixture to a crystallisation temperature within a prescribed range.

Crystalline silica ZSM-23 and its conventional preparation from a reaction mixture containing pyrrolidine directing agent, are taught in US-A-4,076,842. Crystalline silicate ZSM-35 and its conventional preparation from a reaction mixture containing pyrrolidine or ethylenediamine directing agent are taught in US-A-4,088,706.

US-A-4,341,748 shows synthesis of ZSM-5 or ZSM-11 from reaction mixtures comprising, for example, ethanol, ZSM-5 or ZSM-11 seeds, ethanol and seeds, ethanol and ammonium hydroxide, and ethanol, ammonium hydroxide and seeds.

According to the present invention a method of synthesising zeolite ZSM-23 comprises forming an aqueous reaction mixture suitable therefor containing a nitrogenous organic directing agent and sources of silica, alumina and alkali metal cation, maintaining said mixture at 82 to 127°C until nucleation of crystals has commenced, and thereafter maintaining said mixture at 132 to 177°C until crystallisation of said zeolite has occurred. The reaction mixture has the composition, in terms of mole ratios of oxides:

$SiO_2/Al_2O_3$ : 20-200

$H_2O/SiO_2$ : 5-200

$OH^-/SiO_2$ : 0.001-0.3

$K^+/SiO_2$ : 0.001-0.3

$R/SiO_2$ : 0.1-1

wherein R designates organic directing agent and $OH^-$ is calculated only from inorganic sources of alkali.

The organic directing agent may further comprise a 1-6 carbon atom alcohol or diol which constitutes 2 to 60 percent by weight of said reaction mixture.

After nucleation has commenced the reaction mixture is preferably maintained at 143 to 160°C until crystallisation has occurred. Synthesis is facilitated if the reaction mixture additionally comprises 0.01 to 10 percent by weight of seeds of said zeolite. A preferred nitrogenous directing agent is pyrrolidine, and in some cases the sources of silica and alumina are prepared by forming an aqueous gel from sodium silicate, aluminium sulphate and water, mixing seeds of said zeolite with the gel, washing the resultant mixture to remove sodium, and filtering, and then spray-drying, the washed mixture. Optionally thereafter the spray-dried mixture is subjected to ammonium exchange followed by calcination to decompose ammonium cations. Alternatively the source of silica may be prepared by precipitating silica from aqueous sodium silicate by addition of sulphuric acid and washing the precipitated silica with water and aqueous ammonium nitrate to remove sodium.

The reaction mixture has the following composition in favoured embodiments:

|  | Preferred | Most Preferred |
|---|---|---|
| $SiO_2/Al_2O_3$ : | 40-150 | 40-120 |
| $H_2O/SiO_2$ : | 5-50 | 10-40 |
| $OH^-/SiO_2$ : | 0.001-0.2 | 0.001-0.15 |
| $K^+/SiO_2$ . | 0.001-0.3 | 0.001-0.25 |
| $R/SiO_2$ : | 0.2-0.8 | 0.2-0.8 |

and the reaction mixture is maintained at 104 to 121°C until nucleation of crystals has commenced. Advantageously the molar ratio of said nitrogenous organic directing agent to any alcohol or diol which may be present is from 10:1 to 1:5, preferably from 3:1 to 1:3.

The time for which the reaction mixture is maintained until nucleation of crystals has commenced is 6 to 96 hours, preferably 24 to 48 hours. The time for which the mixture is maintained after nucleation and until crystallisation has occurred is 24 to 100 hours.

The invention also comprehends a method of preparing a catalyst for the conversion of organic compounds, which comprises compositing a zeolite synthesised by the method above described with an alumina binder, calcining the composite to decompose organic matter therein (i.e. directing agent or its residue), subjecting the calcined composite to ammonium exchange and calcining the exchanged composite to decompose ammonium ions. Advantageously the resulting composite is subjected to 5-100% steam at at least 300°C for at least one hour at a pressure of 101 to 2500 kPa.

Zeolite ZSM-23 synthesised as herein described, or catalysts comprising it, may be used in the conversion of organic compounds, particularly hydrocarbon conversions such as the alkylation of benzene with ethylene or the dealkylation of ethylbenzene.

Nucleation is confirmed by x-ray detection of a crystal presence greater than (any) seed level. By expiry of the preferred periods of operation at 132 to 177°C crystallisation will be essentially complete.

One aspect of the present invention involves reducing the crystallite size of zeolites. All other things being equal, zeolites of smaller crystallite size tend to have greater initial catalytic activity and tend to maintain activity longer during catalytic processes, particularly with regard to blockage of pores by coke. The effect of crystallite size on zeolite activity is especially pronounced in the case of medium pore size zeolites such as ZSM-23, having unidirectional, nonintersecting channel systems. In accordance with an aspect of the invention, alcohols and/or diols are used in reaction mixtures in order to enable the production of zeolites of reduced crystallite size.

The quantity of $OH^-$ in the above reaction mixtures is calculated only from the inorganic sources of alkali without any organic base contribution. The values of $OH^-/SiO_2$ and $M^+/SiO_2$ brought about by intentionally added reaction mixture components may in fact approach 0. In most cases the reaction mixture will contain at least 0.01 wt.%, preferably at least 0.1 wt.%, and even more preferably from about 1 wt.% to about 10 wt.% seed crystals, and be composed of from about 10 wt.% to about 98 wt.%, preferably from about 30 wt.% to about 90 wt.% solids. It is possible, in accordance with the invention, to synthesise zeolite ZSM-23 from a reaction mixture having a $SiO_2/Al_2O_3$ mole ratio less than 60.

Freshly prepared ZSM-23 usually conforms to the following composition, in terms of mole ratios of oxides and in the anhydrous state:

(0.04 to 0.08)N:(0 to 0.15)$K_2O$:(0.5 to 40)$Al_2O_3$:(100)$SiO_2$ wherein N is nitrogen from nitrogen-containing directing agent.

It is defined by the following x-ray diffraction data:

TABLE I

| Interplanar d-Spacing (Å) (nm x 10) | Relative Intensity (I/I$_o$) |
| --- | --- |
| 11.2 ± 0.23 | Medium |
| 10.1 ± 0.20 | Weak |
| 7.87 ± 0.15 | Weak |
| 5.59 ± 0.10 | Weak |
| 5.44 ± 0.10 | Weak |
| 4.90 ± 0.10 | Weak |
| 4.53 ± 0.10 | Strong |
| 3.90 ± 0.08 | Very Strong |
| 3.72 ± 0.08 | Very Strong |
| 3.62 ± 0.07 | Very Strong |
| 3.54 ± 0.07 | Medium |
| 3.44 ± 0.07 | Strong |
| 3.36 ± 0.07 | Weak |
| 3.16 ± 0.07 | Weak |
| 3.05 ± 0.06 | Weak |
| 2.99 ± 0.06 | Weak |
| 2.85 ± 0.06 | Weak |
| 2.54 ± 0.05 | Medium |
| 2.47 ± 0.05 | Weak |
| 2.40 ± 0.05 | Weak |
| 2.34 ± 0.05 | Weak |

The crystal size of ZSM-23 synthesised by conventional methods is from about 1 to about 2 $\mu$m, whereas that synthesised hereby is within the uniformly narrow size range of from about 0.2 to about 0.5 $\mu$m, usually about 0.2 $\mu$m.

The x-ray diffraction data of Table I were collected with a Philips diffraction system, equipped with a graphite diffracted beam monochromator and scintillation counter, using copper K-alpha radiation. The diffraction data were recorded by step-scanning at 0.04 degrees of two-theta, where theta is the Bragg angle, and a counting time of 4 seconds for each step. The interplanar spacings, d's, were calculated in Ångstrom units (Å) (=0.1 nm), and the relative intensities of the lines, I/I$_o$, where I$_o$ is one-hundreth of the intensity of the strongest line, above background, were derived with the use of a profile fitting routine (or second derivative alogrithm). The intensities are uncorrected for Lorentz and polarization effects. The relative intensities may be given in terms of the symbols vs = very strong (60-100), s = strong (40-60), m = medium (20-40) and w = weak (0-20). It should be understood that diffraction data listed for this sample as single lines may consist of multiple overlapping lines which under certain conditions, such as differences in crystallite sizes or very high experimental resolution or crystallographic changes, may appear as resolved or partially resolved lines. Typically, crystallographic changes can include minor changes in unit cell parameters and/or a change in crystal symmetry, without a change in topology of the structure. These minor effects, including changes in relative intensities, can also occur as a result of differences in cation content, framework composition, nature and degree of pore filling, and thermal and/or hydrothermal history.

The above specified reaction mixture compositions can be prepared utilizing materials which can supply the appropriate component. Such compositions include, when a separate source of aluminium is desired, aluminates or alumina. A preferred source of silicon, which is cost-effective and permits high reaction mixture solids loading, is an amorphous precipitated silica or silica-alumina which has not been dried or calcined, and which contains less than 3 wt.% sodium. It will be understood that each component utilized in the reaction mixture can be supplied by one or more essential reactants and they can be mixed together in any order. The reaction mixture can be prepared either batchwise or continuously.

Other silicon sources, such as dried or calcined silica precipitate, solid silicas or silicas having appreciable, e.g. more than about 3 wt.%, sodium, may be used but are more effective for the synthesis of zeolites other than ZSM-23. For example, a solid silica, e.g. Ultrasil® (a precipitated, spray dried silica) or HiSil® (a precipitated hydrated SiO$_2$ containing about 6 weight percent free H$_2$O and about 4.5 weight percent bound H$_2$O of hydration and having an ultimate particle size of about 0.02 $\mu$m) as the oxide of silicon source favours synthesis of crystal structures such as ZSM-5 or -35 from the above reaction mixture under the temperature programmed conditions required. If sodium silicate is used, ZSM-23 does not nucleate in the range of lower temperature useful herein for nucleation.

Amorphous silica precipitates can be made from a solution of a soluble silica source. Conveniently, the solution is an aqueous solution of a pH ranging from 9 to 12. The source of silica can be any soluble silicate and is preferably sodium silicate. The silica precursor is formed by its continuous precipitation from the solution phase. Accordingly, precipitation comprises initiating precipitation and maintaining said precipitation.

Alteration of the composition of the solution of soluble silica source is undertaken by introducing a precipitating reagent. In one embodiment, the precipitating reagent is a source of acid. Thus, the precipitating reagent can be an acid solution. The acid of the solution may be any mineral acid, such as $H_2SO_4$, HCl, $HNO_3$, etc., and can have a pH ranging from essentially 0 to about 6. Thus, precipitation of the silica precursor can be effected by acid neutralization of a basic solution of a silicate.

The silica can be precipitated alone in the absence of sources of other zeolitic framework elements, e.g. aluminium. In this fashion, both the precipitating reagent and the solution of silica source can be free of intentionally added alumina or alumina source. That is, no aluminium is deliberately added to the silica precipitation reaction mixture, in this embodiment; however, aluminium is ubiquitous and the presence of such a material in minor amounts is due to impurities in the precursors of the reactants or impurities extracted from the reaction vessel. When no source of alumina is added, the amount of alumina in the silica precursor precipitate will be less than about 0.5 weight percent, and generally less than 0.2 weight percent. When a source of alumina is added, the amount of alumina in the silica precursor precipitate will be up to about 5 wt.%. Silica precipitation can be coprecipitation in the presence of soluble sources of other zeolite framework elements including aluminium, gallium, indium, boron, iron and chromium. The soluble source of these other zeolitic framework components can be, for example, nitrates The coprecipitation product would be amorphous, for example an amorphous silica-alumina, silica-boria or silica-gallia.

Continuous precipitation of the amorphous silica precursor may comprise introducing the solution of silica source and the precipitating reagent to a reaction zone while maintaining a molar ratio of silica source to precipitating reagent substantially constant. For example, the precipitating reagent and the silica source are introduced simultaneously into the reaction zone.

The continuous precipitation of silica precursor effects two results. Firstly, silica gel formation is at least substantially eliminated and secondly, precipitated silica precursor particle size exceeds that silica particle size at which silica gel formation is possible. The precipitated silica precursor comprises agglomerated solids in the shape of microspheres. Suspensions of these particles exhibit low viscosities at high solids loading in the subsequent zeolite synthesis reaction mixture of the present invention, even at solids loading equal to or greater than about 10-40%. The particle size of the precipitated silica precursor ranges between 1-500μm, but the average size is 50-100 μm.

Other conditions affecting precipitation of silica precursor include time, pH and temperature. The temperature of the precipitation mixture can range from 80 to 300°F (about 27 to 150°C). The time of contact of the solution of silica source and the precipitating reagent can range from about 10 minutes to several hours at pH maintained from about 6 to 11. Generally, the silica precursor is processed by isolating it, for example by filtration, and removing soluble contaminants therefrom by washing and/or ion exchange. This stage can be considered a solids consolidation step.

The alcohol or diol optionally used in the mixed directing agent contains 1 to 6 carbon atoms, either as straight chain or branched chain alkyls (or alkylenes). Illustrative alcohols or diols include methanol, ethanol, propanol, isopropanol, ethylene glycol, 1,2-propylene glycol, 1,3-propylene glycol or mixtures thereof. The amount of alcohol or diol can range from about 2 to 60% based on the total synthesis mixture weight, e.g., from about 2 to about 10%, more narrowly from about 2 to about 4%, based on the total synthesis mixture weight. It is noted that alcohol or diol addition to the crystallisation reaction mixture may be made alone or in conjunction with the addition of seed crystals of the zeolite sought to be produced.

The original cations, e.g. alkali metal, of zeolites synthesised hereby can be replaced, at least in part, by ion exchange with other cations such as hydrogen, hydrogen ion precursor, and/or metal of Groups IIA, IIIA, IVA, IB, IIB, IIIB, IVB, VIB and/or VIII of the Periodic Table. Organic cations derived from directing agent may be decomposed to hydrogen ions by calcination. Catalytically active forms of the zeolites may include cations of hydrogen, rare earth, aluminium, metals of Groups II and VIII of the Periodic Table, and manganese.

The zeolite may be used as catalyst in a wide variety of organic compound, e.g. hydrocarbon, conversion reactions, and are notably useful in cracking, hydrocracking, dewaxing, wax isomerisation and aromatic compound alkylation, e.g. ethylbenzene synthesis by alkylating benzene with ethylene.

ZSM-23 prepared in accordance with methods described herein can be used either in the as-synthesised form, the alkali metal form and hydrogen form or another univalent of multivalent cationic form.

It can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such components can be exchanged into the composition, impregnated therein or physically intimately admixed therewith. Such components can be impregnated in or on to a zeolite such as, for example, by, in the case of platinum, treating the zeolite with a platinum metal-containing ion. Suitable platinum compounds for this purpose include chloroplatinic acid, platinous chloride and various compounds containing the platinum amine complex. Combinations of metals and methods for their introduction can also be used.

The catalysts may be formed in a wide variety of particle sizes. Generally speaking, the particles can be in the form of a powder, a granule, or a molded product, such as extrudate having particle size sufficient to pass through a 2 mesh (Tyler) screen and be retained on a 400 mesh (Tyler) screen. In cases where the catalyst is molded, such as by extrusion, the crystalline material can be extruded before drying or dried or partially dried and then extruded.

In the case of many catalysts, it is desired to composite the zeolite with another material resistant to the temperatures and other conditions employed in certain organic conversion processes. Such matrix materials include active and inactive materials and synthetic or naturally occurring zeolites as well as inorganic materials such as clays, silica and/or metal oxides, e.g. alumina. The latter may be either naturally occurring or in the form of gelatinous precipitates, sols or gels including mixtures of silica and metal oxides. Use of a material in conjunction with a zeolite, i.e. combined therewith, which is active, may enhance the conversion and/or selectivity of the catalyst in certain organic conversion processes. Inactive materials suitably serve as diluents to control the amount of conversion in a given process so that products can be obtained economically and orderly without employing other means for controlling the rate of reaction. Frequently, crystalline silicate materials have been incorporated into naturally occurring clays, e.g. bentonite and kaolin. These materials, i.e. clays, oxides, etc., function, in part, as binders for the catalyst. It is desirable to provide a catalyst having good crush strength, because in a petroleum refinery the catalyst is often subjected to rough handling, which tends to break the catalyst down into powder like materials which cause problems in processing. Suitable matrix materials and proportions are disclosed in our aforesaid US-A-3,832,449.

In accordance with a particular embodiment of the invention the stability of the zeolite is increased by combining it in the as-synthesised form with an alumina binder, converting the alumina bound zeolite to the hydrogen form and steaming the alumina bound zeolite under conditions sufficient to increase the stability of the catalyst. US-A-4,663,492; 4,594,146; 4,522,929 and 4,429,176 describe conditions for such steam stabilisation of zeolite catalysts. The steam stabilisation step may comprise contacting the alumina bound zeolite with 5-100% steam at a temperature of at least about 300°C (e.g. 300°-650°C) for at least one hour (e.g. 1-200 hours) at a pressure of 101-2,500 kPa. In a more particular embodiment, the catalyst may undergo steaming with 75-100% steam at 315-500°C and atmospheric pressure for 2-25 hours. In accordance with the steam stabilisation treatment described in the above-mentioned US specifications the steaming of the catalyst may take place under conditions sufficient to initially increase the alpha-activity of the catalyst and produce a steamed catalyst having a peak alpha-activity. However, the steaming is not discontinued at this point. Instead the steaming is continued to subsequently reduce the alpha-activity from the peak alpha-activity to an alpha-activity substantially the same as the alpha-activity of the unsteamed catalyst and no more than 25% below the initial alpha-activity of the unsteamed catalyst.

The alpha-test is an indication of the relative catalytic cracking activity of the catalyst compared to a standard catalyst. The value of alpha is the relative rate constant (rate of n-hexane conversion per unit volume of catalyst per unit time). It is based on the activity of highly active silica-alumina cracking catalyst taken as alpha = 1, and is further defined in a letter to the editor, entitled "Superactive Crystalline Alumino-Silicate Hydrocarbon Cracking Catalysts", by P.B. Weisz and J.N. Miale, Journal of Catalysis, Vol. 4, pp. 527-529 (August 1965).

Zeolites ZSM-23 synthesised according to the invention is useful in catalytic dewaxing or hydrodewaxing of heavy oil stocks to provide a catalytically dewaxed oil with reduced wax content; in isomerisation dewaxing of waxy oil feedstock to provide a dewaxed oil product; in catalytic hydrodewaxing of lubricating oil base stocks to provide a catalytically hydrodewaxed lubricating oil base stock with reduced wax content; and in conversion of aromatic compounds to different aromatic compounds by isomerisation, alkylation, disproportionation or transalkylation, as detailed more fully hereinafter.

In general, conversion conditions for these processes include a temperature of from about 100°C to about 800°C, a pressure of from about 0.1 atmosphere (bar) to about 200 atmospheres (203 bar), a weight hourly space velocity of from about 0.08 $hr^{-1}$ to about 2000 $hr^{-1}$ or a liquid hourly space velocity of from about 0.5 $hr^{-1}$ to about 100 $hr^{-1}$, and a hydrogen/feedstock hydrocarbon compound mole ratio of from 0 (no added hydrogen) to about 100.

Cracking hydrocarbons to lower molecular weight hydrocarbons may be effected at a temperature of from about 230°C to about 800°C, a pressure of from about 0.1 atmosphere (bar) to about 35 atmospheres (35.5 bar), a weight hourly space velocity of from about 0.1 $hr^{-1}$ to about 200 $hr^{-1}$ or a liquid hourly space velocity of from about 0.6 $hr^{-1}$ to about 10 $hr^{-1}$, and a hydrogen/feedstock hydrocarbon compound mole ratio of from 0 to about 100.

When the feedstock to the catalytic process comprises a heavy oil stock to be dewaxed, preferred conversion temperature is from about 230°C to about 500°C. When the feedstock comprises a lubricating oil base stock to be dewaxed, preferred conversion temperature is also from about 230°C to about 500°C, with a hydrogen/feedstock lubricating oil base stock mole ratio of from 0 to about 100. For isomerisation dewaxing of such feedstocks, preferred conditions include a temperature of from about 250°F (121°C) to about 500°F (260°C), a pressure of from about 500 psig (35.5 bar) to about 1500 psig (104.5 bar), and a liquid hourly space velocity of from about 0.2 $hr^{-1}$ to about 5.0 $hr^{-1}$.

Feedstock aromatic compounds to be converted include individually and in mixture benzene and monocyclic alkyl-substituted benzenes of from 7 to 12 carbon atoms having up to four methyl and/or ethyl substituents. Non-limiting examples of such feedstock compounds include benzene, toluene, xylene, ethylbenzene, mesitylene (1,3,5-trimethylbenzene), durene (1,2,4,5-tetramethylbenzene), pseudocumene (1,2,4-trimethylbenzene) and mixtures thereof.

Other reactant species may be present, as for alkylation. Alkylating agents include olefins, such as ethylene, propylene and dodecylene, formaldehyde, alkyl halides, alcohols and ethers, the alkyl portion thereof having from 1 to 24 carbon atoms. Numerous other acyclic compounds having at least one reactive alkyl radical may be utilized as alkylating agents.

Products of the aromatic compound conversion process include alkyl-substituted benzene compounds which differ from feedstock compounds depending upon the conversion desired. The following listing presents non-limiting examples:

| Feedstock Aromatic Compounds Include | Other Reactants Include | Product Aromatic Compounds Include |
|---|---|---|
| Benzene | Ethylene | Ethylbenzene |
| Toluene | Methanol | Xylene isomers |
| Xylene isomers, e.g. 9:73:18 wt. ratio of para:meta:ortho | --- | Different Combination of xylene isomers, e.g. 23:57:20 wt. ratio of para:meta:ortho |
| Toluene | --- | Benzene and xylenes |
| Benzene | Propylene | Cumene and diisopropylbenzene |
| Toluene | Propylene | Cymene isomers |

Mechanisms of the aromatic compound conversion process may be isomerisation, alkylation, dealkylation, transalkylation and disproportionation, notably of toluene.

In general, the aromatic conversion processes are conducted at a temperature of from about 150°C to about 760°C, a pressure of from about 0.1 atmosphere (bar) to about 200 atmospheres (203 bar), a weight hourly space velocity of from about 0.1 $hr^{-1}$ to about 2000 $hr^{-1}$ and a hydrogen/feedstock hydrocarbon compound mole ratio of from 0 (no added hydrogen) to about 100.

Such aromatic compound conversion processes include isomerising xylene feedstock components to product enriched in p-xylene with reaction conditions including a temperature from about 150°C to about 600°C, a pressure of from about 0.1 atmosphere to about 70 atmospheres, a weight hourly space velocity of from about 0.1 $hr^{-1}$ to about 200 $hr^{-1}$ and a hydrogen/hydrocarbon mole ratio of from about 0 to about 100; disproportionating toluene to product comprising benzene and xylenes with reaction conditions including a temperature of from about 200°C to about 760°C, a pressure of from about atmospheric to about 65 atmospheres (65.9 bar) and a weight hourly space velocity of from about 0.1 $hr^{-1}$ to about 20 $hr^{-1}$; alkylating aromatic hydrocarbons, e.g. benzene and alkylbenzenes, in the presence of an alkylating agent, e.g. olefins, formaldehyde, alkyl halides and alcohols, with reaction conditions including a temperature of from about 150°C to about 650°C, a pressure of from about atmospheric to about 200 atmospheres, a weight hourly space velocity of from about 2 $hr^{-1}$ to about 2000 $hr^{-1}$ and a feedstock aromatic hydrocarbon/alkylating agent mole ratio of from about 1/1 to about 20/1; and transalkylating aromatic hydrocarbons in the presence of polyalkylaromatic hydrocarbons with reaction conditions including a temperature of from about 200°C to about 760°C, a pressure of from about atmospheric to about 200 atmospheres, a weight hourly space velocity of from about 10 $hr^{-1}$ to about 1000 $hr^{-1}$ and a feedstock aromatic hydrocarbon/ polyalkylaromatic hydrocarbon mole ratio of from about 1/1 to about 16/1.

7

Another particular hydrocarbon conversion reaction in which ZSM-23 synthesised according to the invention can be used as catalyst, is the dealkylation of ethylbenzene. Such a process is of interest in the preparation and separation of para-xylene from a mixture of $C_8$ aromatics (i.e., ethylbenzene plus the three xylene isomers). Suitable conditions for such dealkylation include a reaction temperature of at least 700°F, e.g. 700°F (371°C) to 1000°F (538°C), more particularly, 700-800°F (371-427°C). At lower temperatures of, e.g. about 600°F (316°C) the ethylbenzene may tend to undergo disproportionation rather than dealkylation. The products of ethylbenzene dealkylation reactions are (1) benzene and (2) ethylene and/or ethane. Ethane tends to form in preference to ethylene when hydrogen is introduced into the reaction along with ethylbenzene. The dealkylation reaction may take place, e.g. at low pressure, e.g. atmospheric, in the absence of added hydrogen or at high pressure in the presence of hydrogen, e.g. Octafining conditions. In the latter case, the catalyst may be used in combination with metal such as nickel or platinum having activity for hydrogenation/dehydrogenation reactions. The ethylbenzene dealkylation reaction may take place in the presence or absence of one or more xylene isomers.

In order more fully to illustrate the nature of the invention and the manner of practising same, the following examples are presented.

Example 1 (comparative)

A 130 g quantity of mixed organic directing agent composed of 65 g pyrrolidine and 65 g ethylene glycol was added to a solution containing 30 g $Al_2(SO_4)_3 \cdot 14\ H_2O$, 40 g 88% KOH solution and 900 g water. The resulting solution was added to 395 g of ammonium-form amorphous silica precursor (46% solids) prepared by neutralizing sodium silicate with sulfuric acid and then removing the sodium from the precipitate by washing with water and ammonium nitrate.

A 10 g quantity of ZSM-23 seed crystals was then added to the above mixture to form the reaction mixture for this synthesis. The reaction mixture has a composition, in mole ratios, of:

$SiO_2/Al_2O_3$ = 60
$H_2O/SiO_2$ = 20
$OH^-/SiO_2$ = 0.1
$K^+/SiO_2$ = 0.2
$R/SiO_2$ = 0.62

wherein R is the mixed organic, and a solids content of 13 wt.%. The hydroxide concentration is based on only inorganic sources.

The reaction mixture was then heated directly to 132.2°C and stirred in an autoclave at that temperature for crystallisation. After 168 hours, the resulting crystals were separated from remaining liquid by filtration, washed with water and dried at 121°C overnight.

The resulting crystals proved to be ZSM-23 by analysis.

The ZSM-23 of this example was then mixed with alumina to form a mixture of 65 parts by weight ZSM-23 and 35 parts by weight alumina. Enough water was added to the resulting mixture so that it could be extruded into 1.6 mm (1/16-inch) extrudate. The extrudate was calcined in nitrogen at 538°C for 3 hours, followed by exchange with an aqueous 1.0 N ammonium nitrate solution. The exchanged extrudate was then calcined in air at 538°C for 3 hours.

Example 2 (comparative)

A 130 gram quantity of mixed organic directing agent composed of 65 grams of pyrrolidine and 65 grams of ethylene glycol was added to a solution containing 30 grams of $Al_2(SO_4)_3 \cdot 14H_2O$, 50 grams of 88% KOH solution and 720 grams of water. The resulting solution was added to 650 grams of ammonium-form amorphous silica precursor (39.7% solids) prepared by neutralizing sodium silicate with sulfuric acid and then removing the sodium from the precipitate by washing with water and ammonium nitrate.

A 10 gram quantity of ZSM-23 seed crystals was then added to the above mixture to form the reaction mixture for this synthesis. The reaction mixture had a composition, in mole ratios, of:

$SiO_2/Al_2O_3$ = 82
$H_2O/SiO_2$ = 15
$OH^-/SiO_2$ = 0.1
$K^+/SiO_2$ = 0.2
$R/SiO_2$ = 0.46

wherein R is the mixed organic, and a solids content of 17 wt.%. The hydroxide concentration is based on only inorganic sources.

The reaction mixture was then heated directly to 143.5°C and stirred in an autoclave at that temperature for crystallisation. After 144 hours, the resulting crystals were separated from the remaining liquid by filtration, washed with water and dried at 121°C overnight.

The resulting crystals proved to be ZSM-23 by analysis.

The ZSM-23 of this example was then mixed with alumina to form a mixture of 65 parts by weight ZSM-23 and 35 parts by weight alumina. Enough water was added to the resulting mixture so that it could be extruded into 1.6 mm (1/16-inch) extrudate. The extrudate was calcined in nitrogen at 538°C for 3 hours, followed by exchange with aqueous 1.0 N ammonium nitrate solution. The exchanged extrudate was then calcined in air at 538°C for 3 hours.

Example 3

To demonstrate the improvement of the present invention, a reaction mixture identical to that of Example 1 was initially heated in the stirred autoclave to 121°C and maintained at that temperature far 24 hours while nucleation of ZSM-23 crystals took place. The autoclave temperature was then raised to 143.5°C for crystallisation to be completed. After 260 hours at the crystallisation temperature of 143.5°C, the resulting crystals were separated, washed and dried as in Example 1.

The resulting crystals proved to be ZSM-23 by analysis with a uniform crystal size of about 0.2 $\mu$m.

The ZSM-23 of this example was then made into calcined, exchanged catalyst extrudate exactly as in Example 1.

Example 4

Samples of the Example 1, 2 and 3 catalyst products were individually loaded, in turn, into a fixed bed reactor. They were each contacted with feedstock comprising benzene at 380 cc/hr and ethylene at 160 cc/hr (WHSV of 4 hr$^{-1}$ based on ethylene) at 399°C and 300 psig (21.7 bar) . After 24 hours on stream over each catalyst, the following results were confirmed:

| Catalyst | Ethylene Conversion (wt%) | Product (wt/wt) | | |
|---|---|---|---|---|
| | | Diethylbenzene/ Ethylbenzene | o-Xylene/ Ethylbenzene | Xylenes/ Ethylbenzene |
| Example 1 | 23 | 0.07 | 0.0008 | 0.0008 |
| Example 2 | 22 | 0.00 | 0.003 | 0.008 |
| Example 3 | 81 | 0.14 | 0.0 | 0.0006 |

The superior performance of the material synthesised in Example 3 is evident from the results of Example 4. The Example 3 catalyst converted 81 wt.% of the ethylene, while the Example 1 and 2 catalysts converted only 23 and 22 wt.%. In the alkylation of benzene with ethylene, while desired ethylbenzene is the major product, small amounts of di- and possibly triethylbenzenes are always produced simultaneously with ethylbenzene, such amounts depending on the conversion of benzene to ethylbenzene. The polyethylbenzenes formed can be recycled to the alkylation zone, where they undergo transalkylation with benzene to produce more ethylbenzene. Alternatively, the polyethylbenzenes can be transalkylated with benzene in a separate reactor. The formation of polyethylbenzenes hence does not constitute an ultimate loss of the alkylating agent, ethylene. On the other hand, aromatic compounds other than ethylbenzene and polyethylbenzenes, e.g. xylenes, that are formed during the alkylation reaction, generally referred to as by-products, result in an irreversible loss of ethylene and cause difficulties in the product purification. Production of o-xylene is especially undesirable in view of (1) its relatively low commercial value and (2) the difficulty in separating o-xylene from ethylbenzene by usual methods.

Use of ZSM-23 synthesised according to the invention minimises the production of o-xylene in the alkylation reaction of Example 4. Such use also minimises total xylene by-product, thus minimising required make-up rate to the process.

Claims

1.   A method of synthesising zeolite ZSM-23 which comprises forming a reaction mixture having the composition, in terms of mole ratios of oxides:

$SiO_2/Al_2O_3$ : 20-200
$H_2O/SiO_2$ : 5-200
$OH^-/SiO_2$ : 0.001-0.3
$K^+/SiO_2$ : 0.001-0.3
$R/SiO_2$ : 0.1-1

wherein R designates organic directing agent and $OH^-$ is calculated only from inorganic sources of alkali, maintaining said mixture at 82 to 127°C until nucleation of said crystals has commenced, and thereafter maintaining said mixture at 132 to 177°C until crystallisation of said zeolite has occurred.

2. A method according to claim 1 wherein said organic directing agent further comprises a 1-6 carbon atom alcohol or diol which constitutes 2 to 60 percent by weight of said reaction mixture.

3. A method according to claim 1 or claim 2 wherein, after nucleation has commenced, the reaction mixture is maintained at 143 to 160°C until crystallization has occurred.

4. A method according to any preceding claim wherein the reaction mixture contains from 1 to 10 percent by weight of seeds of said zeolite.

5. A method according to any preceding claim wherein said nitrogenous directing agent is pyrrolidine.

6. A method according to any of claims 1 to 5 wherein said sources of silica and alumina are prepared by forming an aqueous gel from sodium silicate, aluminium sulphate and water, mixing seeds of said zeolite with the gel, washing the resultant mixture to remove sodium, and filtering, and then spray-drying, the washed mixture.

7. A method according to claim 6 in which the spray-dried mixture is subjected to ammonium exchange followed by calcination to decompose ammonium cations.

8. A method according to any of claims 1 to 5 wherein said source of silica is prepared by precipitating silica from aqueous sodium silicate by addition of sulphuric acid and washing the precipitated silica with water and aqueous ammonium nitrate to remove sodium.

9. A method according to any preceding claim wherein said reaction mixture composition is:

$SiO_2/Al_2O_3$ : 40-150
$H_2O/SiO_2$ : 5-50
$OH^-/SiO_2$ : 0.001-0.2
$K^+/SiO_2$ : 0.001-0.3
$R/SiO_2$ : 0.2-0.8

10. A method according to claim 9 wherein said composition is:

$SiO_2/Al_2O_3$ : 40-120
$H_2O/SiO_2$ : 10-40
$OH^-/SiO_2$ : 0.001-0.15
$K^+/SiO_2$ : 0.001-0.25
$R/SiO_2$ : 0.2-0.8

11. A method according to any preceding claim wherein said reaction mixture is maintained at 104 to 121°C until nucleation of crystals has commenced.

12. A method according to any of claims 2 to 11 wherein the molar ratio of said nitrogenous organic directing agent to said alcohol or diol is from 10:1 to 1:5.

13. A method according to claim 12 wherein said ratio is from 3:1 to 1:3.

14. A method according to any of claims 2 to 13 wherein said alcohol or diol is ethanol or ethylene glycol.

15. A method according to any preceding claim wherein the time for which said mixture is maintained until nucleation of crystals has commenced is 6 to 96 hours.

16. A method according to any preceding claim wherein the time for which said mixture is maintained until nucleation of crystals has commenced is 24 to 48 hours.

17. A method according to any preceding claim wherein the time for which said mixture is maintained after nucleation and until crystallisation has occurred is 24 to 300 hours.

18. A method according to any preceding claim wherein the time for which said mixture is maintained after nucleation and until crystallisation has occurred is 24 to 100 hours.

19. A method of preparing a catalyst for the conversion of organic compounds which comprises compositing a zeolite synthesised by the method claimed in any of claims 1 to 23 with an alumina binder, calcining the composite to decompose organic matter therein, subjecting the calcined composite to ammonium exchange and calcining the exchanged composite to decompose ammonium ions.

20. A method according to claim 19 wherein the resulting composite is subjected to 5-100% steam at at least 300°C for at least one hour at a pressure of 101 to 2500 kPa.

21. Use for the conversion of organic compounds of a catalyst comprising zeolite ZSM-23 synthesised by the method claimed in any of claims 1 to 18.

22. Use in accordance with claim 21 wherein the catalyst has been prepared by the method claimed in claim 19 or claim 20.

23. Use according to claims 21 or 22 wherein the conversion is the alkylation of benzene with ethylene.

24. Use according to claim 21 or 22 wherein the conversion is the dealkylation of ethylbenzene.

**Patentansprüche**

1. Verfahren zur Synthese von Zeolith ZSM-23, das die Bildung einer Reaktionsmischung mit einer auf die Molverhältnisse der Oxide bezogenen Zusammensetzung:

$SiO_2/Al_2O_3$ : 20-200
$H_2O/SiO_2$ : 5-200
$OH^-/SiO_2$ : 0,001-0,3
$K^+/SiO_2$ . 0,001-0,3
$R/SiO_2$ : 0,1-1

worin R ein organisches Leitmittel darstellt und OH- nur aus den anorganischen Alkaliquellen berechnet wird, das Halten dieser Mischung bei 82 bis 127°C bis die Keimbildung der Kristalle eingeleitet ist, und das anschließende Halten der Mischung bei 132 bis 177°C umfaßt, bis die Kristallisation des Zeoliths aufgetreten ist.

2. Verfahren nach Anspruch 1, worin das organische Leitmittel außerdem einen Alkohol oder ein Diol mit 1-6 Kohlenstoffatomen umfaßt, das 2 bis 60 Gew.-% der Reaktionsmischung bildet.

3. Verfahren nach Anspruch 1 oder 2, worin die Reaktionsmischung nach Einleitung der Keimbildung bei 143 bis 160°C gehalten wird, bis die Kristallisation aufgetreten ist.

4. Verfahren nach einem der vorstehenden Ansprüche, worin die Reaktionsmischung von 1 bis 10 Gew.-% Kristallkeime des Zeoliths enthält.

5. Verfahren nach einem der vorstehenden Ansprüche, worin das stickstoffhaltige Leitmittel Pyrrolidin ist.

6. Verfahren nach einem der Ansprüche 1 bis 5, worin die Quellen von Siliciumdioxid und Aluminiumoxid hergestellt werden, indem ein wässriges Gel aus Natriumsilikat, Aluminiumsulfat und Wasser gebildet wird, Kristallkeime des Zeoliths mit dem Gel vermischt werden, die resultierende Mischung gewaschen wird, um Natrium zu entfernen, und die gewaschene Mischung filtriert und danach sprühgetrocknet wird.

**7.** Verfahren nach Anspruch 6, worin die sprühgetrocknete Mischung dem Ammoniumaustausch gefolgt von einer Kalzinierung unterzogen wird, um die Ammoniumkationen zu zersetzen.

**8.** Verfahren nach einem der Ansprüche 1 bis 5, worin die Siliciumdioxidquelle hergestellt wird, indem Siliciumdioxid durch Zusatz von Schwefelsäure aus wässrigem Natriumsilikat gefällt wird und das gefällte Siliciumdioxid mit Wasser und wässrigem Ammoniumnitrat gewaschen wird, um das Natrium zu entfernen.

**9.** Verfahren nach einem der vorstehenden Ansprüche, worin die Zusammensetzung der Reaktionsmischung lautet:

$SiO_2/Al_2O_3$ : 40-150
$H_2O/SiO_2$ : 5-50
$OH^-/SiO_2$ : 0,001-0,2
$K^+/SiO_2$ : 0,001-0,3
$R/SiO_2$ . 0,2-0,8

**10.** Verfahren nach Anspruch 9, worin die Zusammensetzung lautet:

$SiO_2/Al_2O_3$ : 40-120
$H_2O/SiO_2$ : 10-40
$OH^-/SiO_2$ : 0,001-0,15
$K^+/SiO_2$ : 0,001-0,25
$R/SiO_2$ : 0,2-0,8

**11.** Verfahren nach einem der vorstehenden Ansprüche, worin die Reaktionsmischung bei 104 bis 121°C gehalten wird, bis die Keimbildung der Kristalle eingeleitet ist.

**12.** Verfahren nach einem der Ansprüche 2 bis 11, worin das Molverhältnis des stickstoffhaltigen organischen Leitmittels zum Alkohol oder Diol von 10:1 bis 1:5 beträgt.

**13.** Verfahren nach Anspruch 12, worin das Verhältnis von 3:1 bis 1:3 beträgt.

**14.** Verfahren nach einem der Ansprüche 2 bis 13, worin der Alkohol oder das Diol Ethanol oder Ethylenglycol ist.

**15.** Verfahren nach einem der vorstehenden Ansprüche, worin die Zeit, während der die Mischung gehalten wird, bis die Keimbildung der Kristalle eingeleitet ist, 6 bis 96 Stunden beträgt.

**16.** Verfahren nach einem der vorstehenden Ansprüche, worin die Zeit, während der die Mischung gehalten wird, bis die Keimbildung der Kristalle eingeleitet ist, 24 bis 48 Stunden beträgt.

**17.** Verfahren nach einem der vorstehenden Ansprüche, worin die Zeit, während der die Mischung nach der Keimbildung und bis die Kristallisation aufgetreten ist, gehalten wird, 24 bis 300 Stunden beträgt.

**18.** Verfahren nach einem der vorstehenden Ansprüche, worin die Zeit, wahrend der die Mischung nach der Keimbildung und bis die Kristallisation aufgetreten ist, gehalten wird, 24 bis 100 Stunden beträgt.

**19.** Verfahren zur Herstellung eines Katalysators zur Umwandlung organischer Verbindungen, welches das Mischen eines Zeoliths, der nach dem Verfahren nach einem der Ansprüche 1 bis 23 hergestellt wurde, mit einem Aluminiumoxidbindemittel, das Kalzinieren des Verbundmaterials, um das organische Material darin zu zersetzen, den Ammoniumaustausch des kalzinierten Verbundmaterials und das Kalzinieren des ausgetauschten Verbundmaterials umfaßt, um die Ammoniumionen zu zersetzen.

**20.** Verfahren nach Anspruch 19, worin das resultierende Verbundmaterial mindestens eine Stunde bei einem Druck von 101 bis 2500 kPa 5-100% Dampf mit mindestens 300°C ausgezetzt wird.

**21.** Verwendung eines Katalysators zur Umwandlung organischer Verbindungen, der Zeolith ZSM-23

EP 0 306 181 B1

umfaßt, der nach dem Verfahren nach einem der Ansprüche 1 bis 18 hergestellt wurde.

**22.** Verwendung nach Anspruch 21, worin der Katalysator nach dem Verfahren nach Anspruch 19 oder Anspruch 20 hergestellt wurde.

**23.** Verwendung nach Anspruch 21 oder 22, worin die Umwandlung die Alkylierung von Benzol mit Ethylen ist.

**24.** Verwendung nach Anspruch 21 oder 22, worin die Umwandlung die Dealkylierung von Ethylbenzol ist.

**Revendications**

**1.** Un procédé de synthèse d'une zéolite ZSM-23 qui comprend la formation d'un mélange réactionnel ayant la composition, en termes de rapports molaires d'oxydes:

$SiO_2/Al_2O_3$ 20-200
$H_2O/SiO_2$ 5-200
$OH\text{-}/SiO_2$ 0,001-0,3
$K^+/SiO_2$ 0,001-0,3
$R/SiO_2$ 0,1-1

dans lesquels R désigne un agent de direction organique et $OH^-$ est calculé sur la base des seules sources inorganiques d'alcalin,
le maintien dudit mélange à une température de 82 à 127°C jusqu'à ce que la nucléation des cristaux ait commencé, et subséquemment le maintien dudit mélange à une température de 132 à 177°C jusqu'à ce que le cristallisation de ladite zéolite ait lieu.

**2.** Un procédé selon la revendication 1, dans lequel ledit agent de direction organique comprend de plus un alcool ou un diol ayant 1 à 6 atomes de carbone qui constitue de 2 à 60% en poids dudit mélange réactionnel.

**3.** Un procédé selon la revendication 1 ou la revendication 2, dans lequel, après que la nucléation ait commencé, le mélange réactionnel est maintenu à une température de 143 à 160°C jusqu'à ce que la cristallisation ait eu lieu.

**4.** Un procédé selon l'une quelconque des revendications précédentes, dans lequel le mélange réactionnel contient de 1 à 10% en poids de germes de ladite zéolite.

**5.** Un procédé selon l'une quelconque des revendications précédentes, dans lequel l'agent de direction azoté est la pyrrolidine.

**6.** Un procédé selon l'une quelconque des revendications 1 à 5, dans lequel lesdites sources du silice et d'alumine sont préparées par formation d'un gel aqueux à partir de silicate de sodium, de sulfate d'aluminium et d'eau, par mélange de germes de ladite zéolite avec le gel, lavage du mélange résultant pour extraire le sodium, et filtration, puis séchage par atomisation du mélange lavé.

**7.** Un procédé selon la revendication 6, dans lequel le mélange séché par atomisation est soumis à un échange à l'ammonium suivi d'une calcination pour décomposer les cations d'ammonium.

**8.** Un procédé selon l'une quelconque des revendications 1 à 5, dans lequel ladite source de silice est préparée par précipitation de silice à partir de silicate de sodium aqueux par addition d'acide sulfurique et lavage de la silice précipitée avec de l'eau et du nitrate d'ammonium aqueux pour extraire le sodium.

**9.** Un procédé selon l'une quelconque des revendications précédentes, dans lequel ledit mélange réactionnel a la composition:

$SiO_2/Al_2O_3$ 40-150
$H_2O/SiO_2$ 5-50
$OH\text{-}/SiO_2$ 0,001-0,2
$K^+/SiO_2$ 0,001-0,3

13

R/SiO$_2$ 0,2-0,8

10. Un procédé selon la revendication 9, dans lequel ladite composition est:
   SiO$_2$/Al$_2$O$_3$ 40-120
   H$_2$O/SiO$_2$ 10-40
   OH-/SiO$_2$ 0,001-0,15
   K$^+$/SiO$_2$ 0,001-0,25
   R/SiO$_2$ 0,2-0,8

11. Un procédé selon l'une quelconque des revendications précédentes, dans lequel ledit mélange réactionnel est maintenu a une température de 104 à 121°C jusqu'à ce que la nucléation des cristaux ait commencé.

12. Un procédé selon l'une quelconque des revendications 2 à 11, dans lequel le rapport molaire dudit agent de direction organique azoté par rapport audit alcool ou diol est de 10/1 à 1/5.

13. Un procédé selon la revendication 12, dans lequel ledit rapport est de 3/1 à 1/3.

14. Un procédé selon l'une quelconque des revendications 2 à 13, dons lequel ledit alcool ou diol est de l'éthanol ou de l'éthylèneglycol.

15. Un procédé selon l'une quelconque des revendications précédentes, dans lequel le temps pendant lequel ledit mélange est maintenu jusqu'à ce que la nucléation des cristaux ait commencé est de 6 à 96 heures.

16. Un procédé selon l'une quelconque des revendications précédentes, dans lequel le temps pendant lequel ledit mélange est maintenu jusqu'à ce que la nucléation des cristaux ait commencé est de 24 à 48 heures.

17. Un procédé selon l'une quelconque des revendications précédentes, dans lequel le temps pendant lequel ledit mélange est maintenu après nucléation et jusqu'à ce que la cristallisation ait lieu est compris entre 24 et 300 heures.

18. Un procédé selon l'une quelconque des revendications précédentes, dans lequel le temps pendant lequel ledit mélange est maintenu après nucléation et jusqu'à ce que la cristallisation ait lieu est compris entre 24 et 100 heures.

19. Un procédé de préparation d'un catalyseur pour la conversion de composés organiques qui comprend la composition d'une zéolite synthétisée par le procédé selon l'une quelconque des revendications 1 à 18 avec un liant à base d'alumine, la calcination du composite pour décomposer les matières organiques comprises dans celui-ci, la soumission du composite calciné à un échange à l'ammonium et la calcination du composite échangé pour décomposer les ions ammonium.

20. Un procédé selon la revendication 19, dans lequel le composite résultant est soumis a 5 à 100% de vapeur à au moins 300°C pendant au moins une heure et sous une pression de 101 à 2500 kPa.

21. Utilisation pour la conversion de composés organiques d'un catalyseur comprenant une zéolite ZSM-23 synthétisée par le procédé selon l'une quelconque des revendications 1 à 18.

22. Utilisation selon la revendication 21, dans laquelle le catalyseur a été préparé par le procédé selon la revendication 19 ou 20.

23. Utilisation selon la revendication 21 ou 22, dans laquelle la conversion est l'alkylation du benzène avec l'éthylène.

24. Utilisation selon la revendication 21 ou 22, dans laquelle la conversion est la désalkylation de l'éthylbenzène.